# DEMANDE DE BREVET EUROPEEN

(11) **EP 0 651 992 A1**
(43) Date de publication de la demande: **10.05.1995**
(21) Numéro de dépôt: 94402373.8
(22) Date de dépôt: 21.10.1994
(51) Int. Cl.: A61K 7/48

(54) **Utilisation de savons comme agents antivieillissement dans des compositions cosmétiques et/ou dermatologiques**

(30) Priorité: 05.11.1993 FR 9313161
(71) Demandeur: L'OREAL, F-75008 Paris (FR)
(72) Inventeur: Koulbanis, Constantin, F-94270 Le Kremlin-Bicetre (FR); Bazin, Roland, F-94400 Vitry-sur-Seine (FR)
(74) Mandataire: Lhoste, Catherine

(57) **Abrégé**

L'invention a pour objet l'utilisation d'un savon formé d'au moins un sel alcalin d'acides gras saturés ou insaturés en tant qu'agent pour lutter contre les signes du vieillissement cutané d'un point de vue cosmétique et/ou dermatologique.

## Description

La présente invention a pour objet l'utilisation de savons en tant qu'agents pour lutter contre les signes du vieillissement de la peau dans des compositions cosmétiques et/ou dermatologiques.

On sait que la couche cornée ou Stratum Corneum est la partie la plus superficielle de l'épiderme. Elle est formée de cellules "cornifiées", non vivantes, appelées cornéocytes. La plupart de ces cornéocytes - qui peuvent être visualisés par des méthodes de coloration - apparaissent, de manière générale, de petite taille (de 750 µm² à 850 µm²) dans une peau jeune et saine.

Dans une peau âgée, au contraire, la majorité des cornéocytes sont de plus grande taille (de 950 µm² à 1000 µm²).

Cette augmentation de taille est liée au ralentissement du renouvellement cellulaire ("turnover"), en raison de l'âge (voir dans "Aging Skin" - Editions Marcel Dekker 1993 - Editeurs J.-L. Lévèque et P.G. Agache, chapitre 14, pp. 199-216, article de P. Corcuff et J.-L. Lévèque).

Par ailleurs, on sait selon l'enseignement de Marks et al. (dans Int. J. Cosmét. Sci., 1981 ; 3, pp. 251-258) qu'une augmentation significative de la taille des cornéocytes se traduit par une diminution importante du taux d'évaporation de l'eau de la surface de la peau, lequel peut être quantifié par la mesure de la valeur de la perte insensible en eau (P.I.E.).

La P.I.E. est donc dans certaines conditions liée au turnover cellulaire. Une augmentation de la P.I.E. est révélatrice d'une activité épidermique plus intense.

Aussi, pour comparer l'efficacité de produits destinés à lutter contre les signes de l'âge, Elias et al. (dans J.A.A.D., 15, n° 4 p 797 et s., 1986) ont suggéré de mesurer les variations de la valeur de la P.I.E. que ces produits induisent lors de leur application répétée sur la peau.

Habituellement, les produits qui sont proposés pour combattre aussi bien les effets du vieillissement "normal" ou physiologique que ceux du vieillissement "provoqué", notamment le vieillissement actinique ou solaire (photovieillissement), peuvent être classés en deux catégories :
- ceux qui agissent en surface par exfoliation ("peeling") des cellules mortes du Stratum Corneum, comme par exemple les alphahydroxyacides,
- ceux qui ont un effet biologique plus direct en profondeur tels que les rétinoïdes.

Cependant l'utilisation de ces produits pose quelques problèmes, en particulier parce qu'ils présentent des effets secondaires reconnus (irritations, risque de tératogénèse).

On constate donc que subsiste dans l'art antérieur le besoin de produits présentant à la fois les effets bénéfiques des substances habituellement destinées au traitement des signes de l'âge et une parfaite sécurité d'emploi.

Ainsi, la demanderesse a maintenant découvert un nouvel effet technique, à savoir un effet anti-vieillissement, lié à l'utilisation sur la peau d'une certaine famille de savons qui, à sa connaissance, n'ont été jusqu'à ce jour utilisés que de manière classique en tant que détersifs.

Cette découverte est à la base de la présente invention.

Il a déjà été proposé dans le document FR-A-2588187 des crèmes contenant de 1 à 3 % de sels alcalins d'acides gras essentiels (A.G.E.) polyinsaturés en C18 à C22, tels que ceux présents dans le savon d'onagre, en mélange avec 10 % de triglycérides d'A.G.E. polyinsaturés provenant d'huile d'onagre.

Selon l'enseignement de ce document, ces crèmes cherchent à améliorer la biodisponibilité au plan cellulaire des A.G.E. qu'elles contiennent, et plus spécialement de l'acide gamma-linoléique présent à des teneurs particulièrement élevées dans l'huile d'onagre, dans le but d'assurer une bonne hydratation de la peau.

La présente invention a, quant à elle, pour objet l'utilisation d'un savon constitué d'au moins un sel alcalin d'acides gras en tant qu'agent pour lutter contre les signes du vieillissement dans, ou pour la fabrication de, une composition cosmétique.

Selon la présente invention, on entend par signe du vieillissement cutané un turnover des cornéocytes ralenti.

L'invention a également pour objet l'utilisation dudit savon dans la préparation d'une composition dermatologique destinée à lutter contre les signes du vieillissement cutané.

Les savons plus particulièrement visés selon l'invention sont constitués, pris seuls ou en mélanges, d'acides gras dont les chaînes hydrocarbonées, qui peuvent être linéaires ou ramifiées, ont un nombre d'atomes de carbone variant entre environ 10 et environ 22, de préférence entre 12 et 18. De préférence, les acides gras sont des acides gras saturés ou monoinsaturés.

Lesdits savons sont également caractérisés par le fait qu'ils contiennent un cation alcalin, de préférence sodium ou potassium, de manière encore plus préférée, du sodium.

Le savon peut en outre contenir une résine telle que la colophane.

Selon un mode préféré de réalisation de l'invention, on utilise un savon tel qu'il peut résulter de la saponification par une base alcaline - en particulier soude ou potasse, de préférence soude - de triglycérides d'acides gras, saturés ou insaturés (provenant par exemple d'huile ou graisse végétale et / ou animale : huile de palme, de coprah, d'olive, d'arachide, de coton, de ricin, de colza et autres, suif de boeuf ou de mouton, graisse de porc) dont les chaînes hydrocarbonées sont essentiellement en C12 - C14 - C16 - C18, et de glycérol (savon du type "savon de Marseille").

Dans le procédé de saponification ci-dessus, la base alcaline est ajoutée en quantité au moins stoechiométrique par rapport à l'acide (neutralisation totale des fonctions acides).

Les quantités d'acides gras (ou acides gras et résine) dans le savon résultant peuvent varier entre 55 % et 70 % en poids, de préférence entre environ 62,5 % et environ 64,5 % en poids, le reste étant constitué de 8 % à 8,5 % en poids d'alcali combiné, de chlorure de sodium et de glycérol, et de 28 % à 29 % en poids d'eau.

La composition de l'invention peut se présenter sous les diverses formes habituellement rencontrées en cosmétologie ou dermatologie telles que pains, gels, crêmes, laits, lotions, mousses et autres.

La composition peut contenir en outre des adjuvants classiques et usuels destinés à la rendre cosmétiquement et/ou dermatologiquement acceptable, lesdits adjuvants étant par exemple choisis, seuls ou en mélange, parmi des agents tensio-actifs de type non-ionique, anionique, cationique ou amphotère, des agents traitants, des ingrédients actifs, des épaississants, des agents de suspension, des agents séquestrants, des agents opacifiants, des colorants, des filtres solaires, des parfums, des excipients, et des conservateurs.

Dans le cadre du procédé selon l'invention, on applique la composition sur la partie du corps à traiter au moins une fois par jour, généralement de une à quatre fois par jour, et ceci pendant plusieurs jours, par exemple de un à cinq jours. Chaque application est suivie d'un rinçage abondant à l'eau.

Un exemple illustrant la présente invention, sans toutefois la limiter, va maintenant être donné.

On donne ci-dessous la méthode suivie pour mesurer la perte insensible en eau (P.I.E., ou TEWL en anglais) :
les P.I.E. des compositions de l'invention ont été mesurées à l'aide d'un évaporimètre (Servomed) qui est un appareil permettant la détermination quantitative de l'évaporation d'eau à partir d'une surface de peau (par exemple de l'avant-bras) selon la méthode décrite par NILSSON GE : Measurement of water exchange through skin, dans Med. Biol. Eng. Comput. 1977 ; 15 : 208 - 18. Des capteurs permettent de mesurer la pression partielle de la vapeur d'eau en deux points situés à des distances différentes de la surface de la peau. On peut déterminer ainsi le gradient de pression partielle de vapeur d'eau entre les deux points, et donc le taux d'évaporation, conformément à la loi de Fick.

### EXEMPLE : Utilisation d'un savon correspondant à la définition du "savon de Marseille"

(i) préparation du savon
   Dans une chaudière, on met de la lessive de soude à faible concentration. On porte à ébullition. On ajoute progressivement la matière grasse en mélangeant continuellement. Après homogénéisation, on fait bouillir et on ajoute de la lessive plus concentrée. L'opération se termine par un relargage au sel (chlorure de sodium) qui permet de séparer le savon de la solution aqueuse contenant entre autres en tant que résidu de saponification, le glycérol.
   Le savon est enfin mis sous la forme de pain à l'aide de procédés connus.
(ii) on applique ledit savon, à l'aide d'un coton humidifié et imprégné pendant cinq secondes dudit savon, à raison de quatre applications par jour, suivies d'un rinçage abondant à l'eau, sur l'avant-bras mouillé de douze personnes, et ceci pendant cinq jours.
(iii) on observe que la P.I.E. augmente en moyenne de 100 % le lendemain du dernier lavage ; elle est toujours augmentée en moyenne de 60 % deux jours après le dernier lavage ; et six jours après, elle reste augmentée de manière étonnante en moyenne de 40 %.

L'exemple ci-dessus montre clairement que les compositions conformes à l'invention augmentent le taux d'évaporation d'eau de la peau (P.I.E.) de manière surprenante, et, par là, qu'elles ont une influence favorable sur le turnover des cellules épidermiques.

## Revendications

1. Utilisation de savons formés de sels alcalins d'acides gras saturés ou insaturés comme agents pour lutter contre les signes du vieillissement cutané.

2. Utilisation selon la revendication 1, caractérisée en ce que lesdits acides gras sont saturés ou monoinsaturés.

3. Utilisation selon la revendication 1 ou 2, caractérisée en ce que lesdits acides gras ont des chaînes hydrocarbonées ayant un nombre d'atomes de carbone choisi de 10 à 22.

4. Utilisation selon la revendication 3, caractérisée en ce que le nombre d'atomes de carbone est choisi de 12 à 18.

5. Utilisation selon l'une quelconque des revendications précédentes, caractérisée en ce que les savons contiennent un sel de métal alcalin choisi parmi le sodium et le potassium.

6. Utilisation selon la revendication 5, caractérisée en ce que les savons contiennent du chlorure de sodium.

7. Utilisation selon l'une quelconque des revendications précédentes, caractérisée en ce que les savons contiennent en outre une résine.

8. Utilisation selon l'une quelconque des revendications précédentes, caractérisée en ce que les savons résultent de la saponification par une base alcaline de triglycérides d'acides gras saturés ou insaturés et de glycérol.

9. Utilisation selon la revendication 8, caractérisée en ce que lesdits acides gras ont des chaînes hydrocarbonées ayant un nombre de carbone choisi de 10 à 22, de préférence 12 à 18.

10. Utilisation selon l'une quelconque des revendications précédentes, caractérisée en ce que les savons contiennent de 62,5 % à 64,5 % en poids d'acides gras ou d'acides gras et de résine, de 8 % à 8,5 % en poids d'un mélange de soude, chlorure de sodium et de glycérol et de 28 % à 29 % en poids d'eau.

11. Utilisation selon l'une quelconque des revendications précédentes, caractérisée en ce que ledit savon contient en outre des adjuvants, lesdits adjuvants étant choisis, seuls ou en mélange, parmi des agents tensio-actifs de type non-ionique, anionique, cationique ou amphotère, des agents traitants, des ingrédients actifs, des épaississants, des agents de suspension, des agents séquestrants, des agents opacifiants, des colorants, des filtres solaires, des parfums, des excipients, et des conservateurs.

12. Utilisation de savons formés de sels alcalins d'acides gras saturés ou insaturés tels que définis à l'une quelconque des revendications précédentes, pour la préparation d'une composition dermatologique destinée à lutter contre les signes du vieillissement cutané.
